(19) Europäisches Patentamt
European Patent Office
Office européen des brevets

(11) EP 1 473 304 A1

(12) **EUROPÄISCHE PATENTANMELDUNG**

(43) Veröffentlichungstag:
**03.11.2004 Patentblatt 2004/45**

(51) Int Cl.[7]: **C07K 14/52**, C12N 15/19, A61K 38/19, A61P 9/00

(21) Anmeldenummer: **03010014.3**

(22) Anmeldetag: **02.05.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(71) Anmelder: **BOEHRINGER INGELHEIM PHARMA GMBH & CO. KG**
**55216 Ingelheim am Rhein (DE)**

(72) Erfinder:
- **Seidler, Randolph**
  **Sandy Hook, CT 06482 (US)**
- **Lenter, Martin**
  **89073 Ulm (DE)**
- **Doods, Henri**
  **88447 Warthausen (DE)**
- **Wandl, Robert**
  **1030 Wien (AT)**
- **Necina, Roman**
  **1210 Wien (AT)**

(54) **Verfahren zur Herstellung eines N-terminal modifizierten chemotaktischen Faktors**

(57) Die Erfindung betrifft ein Verfahren zur Herstellung von pyroGlu-MCP-1 aus rekombinant hergestelltem Gln-MCP-1, bei dem Gln-MCP-1 bei einer Temperatur im Bereich von 30°C und 80°C in einer Pufferlösung mit einer Salzkonzentration im Bereich von 10 mM bis 160 mM und einem pH-Wert im Bereich von 2 bis 7.5 inkubiert wird, bis mindestens 90 % des MCP-1 in Form des pyroGlu-MCP-1 vorliegen.



Printed by Jouve, 75001 PARIS (FR)

## Beschreibung

Die Erfindung betrifft ein Verfahren zur Herstellung von pyroGlu-MCP-1 aus rekombinant hergestelltem Gln-MCP-1 sowie Zusammensetzungen enthaltend pyroGlu-MCP-1-Präparationen.

MCP-1 (monocyte chemoattractant protein-1; auch bekannt unter den Bezeichnungen: monocyte chemotactic and activating factor 'MCAF', macrophage chemotactic factor 'MCF', tumor necrosis factor stimulated gene-8 'TSG-8', ‚HC-11', smooth muscle cell chemotactic factor 'SMC-CF', lymphocyte derived chemotactic factor'LDCF' sowie glioma derived chemotactic factor 'GDCF') ist ein Mitglied der CC-Chemokin-Familie. Das MCP-1-Protein des Menschen wurde ursprünglich im US-Patent Nr. 5,714,578 beschrieben. Es wird unter natürlichen Bedingungen im Organismus (nativ) als 99 Aminosäuren langes Vorläuferprotein synthetisiert, welches anschließend zu einem Peptid mit 76 Aminosäureresten prozessiert wird. Gereiftes humanes MCP-1 (hMCP-1) ist ein Glycoprotein mit einem Molekulargewicht von 14 kDa und wird von vielerlei Zellarten, z.B. glatten Gefäßmuskelzellen und Endothelzellen sekretiert (Leonard und Yoshimura (1990), Immology Today 11, 97 - 101). Es enthält zwei intramolekulare Disulfidbrücken und ist bei nativer Expression O-glykosyliert und sialylyliert (J. Yan Ling et al. (1990), Journal ofBiological Chemistry, 265, 18318 - 18321).

Das Protein ist ein Produkt des JE-Gens der chromosomalen Lokalisation 17q11.2-q21.1. Dieser Genlocus ist auch bekannt als SCY A2 ("small inducible cytokine A2). Das menschliche Gen wurde zuerst beschrieben im US-Patent Nr. 5,212,073. Die Expression dieses Gens kann durch vielerlei Zytokine, so z.B. Tumor necrosis factor alpha, aber auch z.B. durch Immunglobulin G induziert werden. Die Gensequenz und weitere Informationen über das Gen und das Genprodukt sind in der NCBI-Datenbank unter der Zugangsnummer M37719 zugänglich (s. Tabelle 1).

Tabelle 1:

| Human monocyte chemotactic protein gene, complete cds |
|---|

```
LOCUS HUMMCHEMP    2776 bp    DNA      linear    PRI 13-MAY-1994
DEFINITION  Human monocyte chemotactic protein gene, complete cds.
ACCESSION   M37719
VERSION     M37719.1  GI:187447
KEYWORDS    monocyte chemotactic protein.
SOURCE      Homo sapiens (human)
ORGANISM    Homo sapiens
            Eukaryota; Metazoa; Chordata; Craniata; Vertebrata;
            Euteleostomi; Mammalia; Eutheria; Primates; Catarrhini;
            Hominidae; Homo.
REFERENCE   1  (bases 1 to 2776)
AUTHORS     Shyy,Y.J., Li,Y.S. and Kolattukudy,P.E.
TITLE       Structure of human monocyte chemotactic protein gene and its
            regulation by TPA
JOURNAL     Biochem. Biophys. Res. Commun. 169 (2), 346-351 (1990)
MEDLINE     90290466
COMMENT     Original source text: Human DNA.
FEATURES             Location/Qualifiers
     source          1..2776
                     /organism="Homo sapiens"
                     /db_xref="taxon:9606"
     gene            598..2080
                     /gene="SCYA2"
     CDS             join(598..673,1472..1589,1975..2080)
                     /gene="SCYA2"
                     /note="monocyte chemotactic protein"
                     /codon_start=1
                     /protein_id="AAA18102.1"
                     /db_xref="GI:487124"
     translation=    "MKVSAALLCLLLIAATFIPQGLAQPDAINAPVTCCYNFTNRKISVQRLA
                      SYRRITSSKCPKEAVIFKTIVAKEICADPKQKWVQDSMDHLDKQTQTPKT"
     exon            <598..673
                     /gene="SCYA2"
                     /note="monocyte chemotactic protein"
                     /number=1
     exon            598..673
                     /gene="SCYA2"
                     /note="monocyte chemotactic protein"
                     /number=1
     intron          674..1471
                     /gene="SCYA2"
                     /note="monocyte chemotactic protein intron A"
     exon            1472..1589
                     /gene="SCYA2"
                     /note="monocyte chemotactic protein"
                     /number=2
     intron          1590..1974
                     /gene="SCYA2"
                     /note="monocyte chemotactic protein intron B"
     exon            1975..2080
                     /gene="SCYA2"
                     /note="monocyte chemotactic protein"
                     /number=3
     exon            1975..>2080
                     /gene="SCYA2"
                     /note="monocyte chemotactic protein"
                     /number=3
```

```
BASE COUNT      700 a    727 c    565 g    781 t      3 others
ORIGIN
1    cagttcaatg tttacacaat cctacagttc tgctaggctt ctatgatgct actattctgc
61   atttgaatga gcaaatggat ttaatgcatt gtcagggagc cggccaaagc ttgagagctc
121  cttcctggct gggaggcccc ttggaatgtg gcctgaaggt aagctggcag cgagcctgac
181  atgctttcat ctagtttcct cgcttccttc cttttcctgc agttttcgct tcagagaaag
241  cagaatcctt aaaaataacc ctcttagttc acatctgtgg tcagtctggg cttaatggca
301  ccccatcctc cccatttgcg tcatttggtc tcagcagtga atggaaaaaa gtgctcgtcc
361  tcaccccccct gcttcccttt cctacttcct ggaaatccac aggatgctgc atttgctcag
421  cagatttaac agcccactta tcactcatgg aagatccctc ctcctgcttg actccgccct
481  ctctccctct gcccgctttc aataagaggc agagacagca gccagaggaa ccgagaggct
541  gagactaacc cagaaacatc caattctcaa actgaagctc gcactctcgc ctccagcatg
601  aaagtctctg ccgccccttct gtgcctgctg ctcatagcag ccaccttcat tccccaaggg
661  ctcgctcagc caggtaaggc cccctcttct tctccttgaa ccacattgtc ttctctctga
721  gttatcatgg accatccaag cagacgtggt acccacagtc ttgctttaac gctacttttc
781  caagataagg tgactcagaa aaggacaagg ggtgagcccc aaccacacag ctgctgctcg
841  gcagagcctg aactagaatt ccagctgtga acccaaatcc agctccttcc aggattcagg
901  atccagctct gggaacacac tcagcagtta ctccccccagc tgcttccagc agagtttggg
961  gatcagggta atcaaagaga agggtgggtg tgtaggctgt ttccagacac gctggagacc
1021 cagaatctgg tctgtgcttc attcacctta gcttccagag accggtgact ctgcaggtaa
1081 tgagtatcag ggaaactcat gaccaggcat agctattcag agtctaaaag gaggctcata
1141 gtggggctcc cagctgatct tccctggtgc tgatcatctg gattattggt ccgtcttaat
1201 gacacttgta ggcattatct agctttaaca gctcctcctt ctctctgtcc attatcaatg
1261 ttatataccc cattttacag cataggaaac tgagtcattg ggtcaaagat cacattctag
1321 ctctgaggta taggcagaag cactgggatt taatgagctc tttctcttct cctgcctgcc
1381 ttttgttttt tcctcatgac tcttttctgc tcttaagatc agaataatcc agttcatcct
1441 aaaatgcttt tctttgtggt ttattttcca gatgcaatca atgccccagt cacctgctgc
1501 tataacttca ccaataggaa gatctcagtg cagaggctcg cgagctatag aagaatcacc
1561 agcagcaagt gtcccaaaga agctgtgatg tgagttcagc acaccaacct tccctggcct
1621 gaagttcttc cttgtggagc aagggacaag cctcataaac ctagagtcag agagtgcact
1681 atttaactta atgtacaaag gttcccaatg ggaaaactga ggcaccaagg gaaaaagtga
1741 accccaacat cactctccac ctgggtgcct attcagaaca ccccaatttc tttagcttga
1801 agtcaggatg gctccacctg gacacctata ggagcagttt gccctgggtt ccctccttcc
1861 acctgcgtcc tcctagtctc catggcagct cgcttttggt gcagaatggg ctgcacttct
1921 agaccaaaac tgcaaaggaa cttcatctaa ctctgtctcc tcccttcccc acagcttcaa
1981 gaccattgtg gccaaggaga tctgtgctga ccccaagcag aagtgggttc aggattccat
2041 ggaccacctg gacaagcaaa cccaaactcc gaagacttga acactcactc cacaacccaa
2101 gaatctgcag ctaacttatt ttcccctagc tttccccaga caccctgttt tattttatta
2161 taatgaattt tgtttgttga tgtgaaacat tatgccttaa gtaatgttaa ttcttattta
2221 agttattgat gttttaagtt tatctttcat ggtactagtg tttttttagat acagagactt
2281 ggggaaattg cttttcctct tgaaccacag ttctacccct gggatgtttt gagggtcttt
2341 gcaagaatca ttaatacaaa gaattttttt taacattcca atgcattgct aaaatattat
2401 tgtggaaatg aatattttgt aactattaca ccaaataaat atattttttgt acaaaaacctg
2461 acttccagtg ttttcttgaa ggaaattaca aagctgagag tatgagcttg gtggtgacaa
2521 aggaacatga tttcagaggg tggggcttac attttgaagg aatgggaaag tggattggcc
2581 cnntntcttc ctccactggg tggtctcctc tgagtctccg gtagaagaat ctttatggca
2641 ggccagttag gcattaaagc accacccttc cagtcttcaa cataagcagc ccagagtcca
2701 atgaccctgg tcacccattt gcaagagccc acccccattt cttttgctct cacgaccctg
2761 accctgcatg caattt
//
```

In der oben erwähnten US 5,714,578 wurde bereits beschrieben, dass im Falle eines aus nativer Quelle isolierten MCP-1 Proteins der N-Terminus blockiert ist. Wie erst später gefunden wurde, ist dies zurückzuführen auf eine posttranslationale Modifikation, bei der das nach Abspaltung der Leadersequenz am N-Terminus des reifen Proteins freigelegte Glutamin unter Austritt eines Moleküls $NH_3$ zu einem pyroGlutamatrest zyklisiert.

Die Identifizierung des für MCP-1 codierenden open reading frames in der US 5,212,073 ermöglichte die rekombinante Expression des Proteins. Ein auf rekombinantem Weg z.B. in *E. coli* hergestelltes MCP-1 weist nicht die typischen N- bzw. O-Glykosylierungsmuster des nativen MCP-1 auf. Eine auf diesem Wege hergestellte MCP-1-Präparation widersetzt sich auch anders als eine aus nativem Material gewonnene Präparation nicht dem Edman-Abbau, enthält also unblockierte N-Termini (Glutamine). In zellulären Assays, die auf der chemotaktischen Wirkung auf Makrophagen beruhten, konnte ursprünglich kein Unterschied zwischen der biologischen Aktivität nativer und auf rekombinantem Weg gewonnener MCP-1-Präparationen festgestellt werden.

MCP-1 wirkt unter physiologischen Bedingungen als Agonist an den Beta-Chemokin-Rezeptoren CCR2 und CCR4, die beide hauptsächlich auf Monozyten exprimiert sind und außerdem auch noch auf Basophilen sowie auf T- und B-Lymphozyten gefunden wurden. MCP-1 induziert die Monozytenchemotaxis bereits in subnanomolaren Konzentrationen. Die Rezeptoren CCR2 und CCR4 sind G-Protein-gekoppelte Sieben-Transmembrandomänen-Rezeptoren, die zur Aktivierung von Monozyten und verstärkter Adhesion von Integrinen führen. Dieser Prozess resultiert letztlich in einem Andocken von Monozyten an Endothelzellen und anschließendem Austreten der Monozyten aus dem Gefäßsystem.

In der WO 98/44953 ist ein Einfluss von MCP-1 auf die Arteriogenese, also das Wachstum von Kollateralarterien und/oder anderen Arterien ausgehend von bereits existierenden arteriolären Verbindungen offenbart. Auf der Basis dieser Erkenntnis wurde in der genannten internationalen Patentanmeldung vorgeschlagen, MCP-1 zu therapeutischen Zwecken einzusetzen, nämlich zur Behandlung von Gefäßverschlusserkrankungen, die durch die Anregung von Gefäßneubildungen gelindert werden können. Solche Gefäßverschlusserkrankungen sind insbesondere Koronararterienerkrankungen (coronary artery disease, CAD), periphere Arterienverschlusserkrankungen (peripheral arterial occlusive disease, PAOD), zerebrale und mesenteriale arteriellen Verschlusserkrankheiten, etc. Außerdem wurde darin vorgeschlagen, MCP-1-neutralisierende Agenzien, wie z.B.

Anti-MCP-1-Antikörper zur Verhinderung von Gefäßneubildungen einzusetzen um damit insbesondere das Wachstums von Tumoren zu bekämpfen, das auf eine ausreichende Blutversorgung und damit eine hinreichende Vaskularisierung des Tumorgewebes angewiesen ist.

Um das MCP-1-Protein wie oben erläutert in einem therapeutischen Ansatz zur Förderung der Vaskularisierung von Geweben verwenden zu können, müssen hinreichende Mengen dieses Proteins in für pharmazeutische Zwekke hinreichend hoher Reinheit bereitgestellt werden. Humanes MCP-1 wurde ursprünglich in nativer Form aus Kulturen der menschlichen Gliomzelllinie U105MG oder aus menschlichen mononucleären Leukozyten des peripheren Blutes gewonnen. Eine Isolierung von für therapeutische Zwecke gedachtem Protein aus diesen Quellen scheitert bereits daran, dass auf diese Weise hinreichend große Mengen allenfalls unter inakzeptabel hohem Aufwand und/oder Materialeinsatz zu gewinnen wäre: Menschliche Leukozyten wären in der erforderlichen Menge nicht zu erhalten. Die Gliomzellen ließen sich zwar fast unbeschränkt vermehren, eignen sich jedoch nicht zur Kultivierung in biotechnologischen Fermentern und erfordern darüber hinaus z.B. fötales Kälberserum zu deren Kultivierung, mit allen damit verbundenen Problemen.

Anstelle der nativen Expression von MCP-1 bietet sich daher an, MCP-1 auf rekombinantem Weg herzustellen. In der Tat ist rekombinantes humanes MCP-1 bereits auf dem Markt erhältlich, so insbesondere von den Firmen R&D Systems (Katalog-Nr. 279-MC) und Peprotech (Katalog-Nr. 300-04; s. Tabelle 2; die angegebenen Katalog-Nummern sind auf dem Stand Januar 2003). Gemäß Produktspezifikation der käuflich erhältlichen rekombinanten MCP-1-Präparationen enthält das darin enthaltene MCP-1-Protein die Aminosäure Glutamin (Q) am N-Terminus. Die Produktbeschreibung deutet außerdem darauf hin, dass diese Proteinpräparation insbesondere in rekonstituiertem, flüssigen Zustand sehr empfindlich ist (Lagerung bei 4°C empfohlen für max. 1 Woche). In der Tat wurde bei Versuchen der Erfinder (*interner* Stand der Technik) festgestellt, dass bei einer Lagerung der konventionellen MCP-1-Proteinpräparationen in gelöstem Zustand die - biologisch aktive - Proteinpräparation insbesondere bei Temperaturen über 4 °C sehr schnell Zeichen von Verunreinigung durch Abbauprodukte zu zeigen scheint (Auftreten von Nebenbanden in analytischen HPLC-Chromatogrammen).

Tabelle 2

| (Auszug aus der Internet-Website der Firma Peprotech http://www.peprotech.com/content/details.htm?results=1&prod=1392): | |
|---|---|
| **Recombinant Human MCAF (Human MCP-1)** | |
| **Description** | Human monocyte chemotactic protein-1 (MCP-1) also known as macrophage/monocyte chemotactic and activating factor (MCAF) is an 8.6 kDa protein containing 76 amino acid residues. It plays an important role in the inflammatory response of blood monocytes and tissue macrophages. |
| **Catalog #** | 300-04 |

Tabelle 2 (fortgesetzt)

| (Auszug aus der Internet-Website der Firma Peprotech http://www.peprotech.com/content/details.htm?results=1&prod=1392): | |
|---|---|
| **Recombinant Human MCAF (Human MCP-1)** | |
| **Source** | E.coli |
| **Formulation** | The sterile filtered solution was lyophilized with no additives. |
| **Stability** | The lyophilized protein is stable for a few weeks at room temperature, but best stored at -20°C. Reconstituted human MCAF should be stored in working aliquots at -20°C. |
| **Purity** | Greater than 99% by SDS-PAGE and HPLC analyses. Endotoxin level is less than 0.1 ng per µg (1EU/µg). |
| **Reconstitution** | We recommend a quick spin followed by reconsitution in water to a concentration of 0.1-1.0 mg/ml. This solution can then be diluted into other aqueous buffers and stored at 4°C for 1 week or -20°C for future use. |
| **Biological Activity** | Determined by its ability to chemoattract human monocytes using a concentration range of 5.0-20.0 ng/ml. |
| **AA Sequence** | QPDAINAPVT CCYNFTNRKI SVQRLASYRR ITSSKCPKEA VIFKTIVAKE ICADPκQKWV QDSMDHLDKQ TQTPKT |
| **Country of Origin** | USA |

In Van Coillie et al. (1998), Biochemistry 37: 12672, 12673 a.E. ist im Zusammenhang mit Versuchen zum Einfluss N-terminaler Modifikationen auf die biologische Aktivität von MCP-2 ein Verfahren beschrieben, bei dem eine auf rekombinantem Weg erhaltene MCP-2-Präparation in 0.01 M $Na_2HPO_4$ pH 8.0, 24 Stunden bei 37 °C inkubiert wird. MCP-2 weist auf der Ebene der Aminosäuresequenz einen Homologiegrad von 62 % zu MCP-1 auf und unterscheidet sich von diesem auch insoweit als N-terminal unmodifiziertes MCP-1 im Gegensatz zu MCP-2 biologisch aktiv ist (vgl. Zeile "Biological Activity" in Tabelle 2). Das Ausmaß der unter den o.g. Bedingungen erfolgenden Konvertierung des N-terminalen Glutamin-Rests zu einem pyroGlutamat-Rest wurde von den Autoren nicht bestimmt. Eine Anwendung vergleichbarer Bedingungen auf MCP-1 führt nach Erkenntnis der Erfinder (*interner* Stand der Technik) jedenfalls nur zu einer partiellen Zyklisierung der N-terminalen Aminosäure (vgl. Tabelle 3, Vergleichsversuch 2).

Eine Aufgabe der Erfindung ist in Anbetracht der vorstehenden Erläuterungen daher die Bereitstellung eines Verfahrens, mit dessen Hilfe eine MCP-1-Präparation (MCP-1-Zusammensetzung) hergestellt werden kann, die sich (a) hinsichtlich ihrer biologischen Aktivität für therapeutische Zwecke eignet und die (b) Vorteile hinsichtlich arzneimittelrechtlicher Zulassungsverfahren bietet, welche im Falle von Biopharmazeutika besonders schwer zu erfüllende Anforderungen z.B. hinsichtlich der Reinheit und Reproduzierbarkeit der Herstellung des Arzneimittels beinhalten, und die insbesondere (c) eine hohe Lagerstabilität aufweist. Eine damit im Zusammenhang stehende weitere Aufgabe ist die Bereitstellung von Zusammensetzungen bzw. Präparationen, die auf rekombinantem Weg hergestelltes MCP-1 enthalten und sich zu den vorgenannten Zwecken eignen bzw. die vorgenannten Vorteile bieten.

Diese Aufgaben werden gelöst durch die in den Ansprüchen angegebenen Verfahren und MCP-1 enthaltenden Zusammensetzungen.

So wird erfindungsgemäß ein Verfahren zur Herstellung von pyroGlu-MCP-1 aus rekombinant hergestelltem Gln-MCP-1 bereitgestellt, bei dem Gln-MCP-1 bei einer Temperatur im Bereich von 30°C und 80°C, vorzugsweise im Bereich von 30°C und 70°C und weiter vorzugsweise im Bereich von 35 °C bis 60 °C, in einer Pufferlösung inkubiert wird, die eine Salzkonzentration im Bereich von 10 mM bis 160 mM, vorzugsweise im Bereich von 10 mM bis 100 mM, aufweist und die einen pH-Wert im Bereich von 2 bis 7,5, vorzugsweise 3,5 bis 7,5, weiter vorzugsweise 3,5 bis 6,5, weiter vorzugsweise 5 bis 6,5, weiter vorzugsweise 5,5 bis 6,5 und weiter vorzugsweise einen pH-Wert von um die 6 aufweist. Die Inkubation wird dabei so lange durchgeführt, bis mindestens 90 %, vorzugsweise mindestens 95 % und weiter vorzugsweise mindestens 96 % des in der Inkubationspufferlösung enthaltenen MCP-1 in Form des pyroGlu-MCP-1 vorliegen.

Unter „MCP-1" wird im Rahmen dieser Offenbarung das MCP-1-Protein (ohne Präprosequenz), je nach Kontext mit N-terminalem Glutamin-Rest ("Gln-MCP-1"; vgl. die in Tabelle 1 und Tabelle 2 gezeigte Aminosäuresequenz) oder mit bereits zum pyroGlutamat-Rest konvertiertem/zyklisiertem N-Terminus ("pyroGlu-MCP-1") verstanden. Es ist jedoch einsichtig, dass Modifikationen des MCP-1-Proteins, die sich nicht auf dessen biologische Funktion auswirken (insb. die Monozyten-anziehende Wirkung oder die Wirkung am CCR-Rezeptor) und nicht dessen Struktur so abändern, dass die oben beschriebenen Reaktionsparameter nicht mehr zum gewünschten Ergebnis führen (sich das Protein also nicht mehr nach dem erfindungsgemäßen Verfahren in eine pyroGlu-Variante überführen lässt), nicht aus dem Schutzbereich herausführen. So ist das erfindungsgemäße Verfahren natürlich auch anwendbar auf ein MCP-1, bei dem ein konservativer Aminosäureaustausch, so z.B. Serin zu Threonin oder Leucin zu Isoleucin, stattgefunden hat, soweit sich dieser weder auf die biologische Funktion oder Aktivität des resultierenden Proteins, noch auf die Konvertierbarkeit des N-terminalen Glutamins zu pyroGlutamat gemäß dem obigen Verfahren auswirkt. Das erfindungsgemäße Verfahren lässt sich auch auf MCP-1-Proteine anderer Säuger wie z.B. der Ratte, der Maus, des Meerschweinchens, des Kaninchens und des Frettchens (und einiger mehr) anwenden.

Die Pufferlösung, in der das vorstehend beschriebene Verfahren ausgeführt wird, ist vorzugsweise eine phosphatgepufferte (Natrium- und/oder Kaliumphosphat-gepufferte) wässrige Lösung niedriger oder physiologischer Molarität, nämlich im Bereich von 10 bis 160 mM, 10 bis 80 mM, 10 bis 50 mM, 20 bis 40 mM oder um die 20 oder 40 mM. Nimmt man längere Inkubationszeiten in Kauf, so kann entsprechend einer speziellen Ausführungsform der Erfindung auch bei physiologischen Molaritäten, wie z.B. um die 150 mM Salzkonzentration, gearbeitet werden, wobei diese Molarität vorzugsweise durch Verwendung einer phosphatgepufferten Salinelösung oder "PBS" erzielt wird. Der Nachteil der längeren Inkubationsdauer - und damit der Gefahr eines steigenden Anteils an Abbau-, Neben- oder Oxidationsprodukten - wird bei dieser speziellen Ausführungsform durch den Vorteil wettgemacht, die Proteinpräparation gleich in Form einer Lösung mit physiologischer Salzkonzentration gewinnen zu können.

Die Pufferlösung, in der der Modifikationsschritt ausgeführt wird, kann darüber hinaus auch noch z.B. ein (mildes) Detergens, ein Antioxidationsmittel, ein Konservierungsmittel einen Komplexbildner, Stabilisatoren, antimikrobielle Reagenzien, etc. enthalten.

Die Inkubationstemperatur wird im Bereich von 30 °C bis 80 °C gewählt, also über Raumtemperatur. Bei niedrigeren Temperaturen verläuft der Konvertierungsschritt nur langsam. Um dennoch eine praktisch vollständige Konvertierung zu erreichen, müsste dann die Inkubationsdauer erheblich verlängert werden auf über eine Woche. Dies würde zu im biotechnologischen Verfahrensablauf inakzeptablen Standzeiten führen und außerdem die Gefahr anderweitiger Verunreinigungen der Proteinlösung (durch Abbauoder Oxidationsprodukte, durch Bakterien, Viren und sonstige Keime) erheblich erhöhen. Andererseits führt eine Erhöhung der Inkubationstemperatur auf über 80 °C zwar zu einer starken Beschleunigung der Konvertierungsreaktion, dabei aber auch zu einer stärkeren Bildung unerwünschter Neben- und Abbauprodukte (vgl. Beispiele 1 und 2). Sind Standzeiten von bis zu 6 Tagen akzeptabel, dann ist eine Inkubationstemperatur von 35 bis 40 °C besonders bevorzugt. In Fällen, in denen nach Möglichkeit deutlich kürzer inkubiert werden soll, kann dagegen auch im Bereich z.B. von 50 bis 60, 70 oder 80 °C inkubiert werden. Temperaturen zwischen 40 und 50 °C führen natürlich auch zum gewünschten Ergebnis.

Der pH-Wert der Inkubationspufferlösung sollte im Bereich von 2 und 7,5 liegen, also im neutralen bis sauren Bereich. Vorzugsweise liegt er im Bereich von 3,5 bis 7,5. weiter vorzugsweise im Bereich von 3,5 bis 6,5, weiter vorzugsweise im Bereich von 5 bis 6,5, weiter vorzugsweise im Bereich von 5,5 bis 6,5 und weiter vorzugsweise wählt man einen pH-Wert von um die 6.

Bei geeigneter, z.B. in den Beispielen näher beschriebener Wahl der obigen Parameter kann die pyroGlu-MCP-1-Variante in einem Reinheitsgrad von jedenfalls 90 %, aber auch höher, z.B. 95%, 96% oder 98% gewonnen werden, wobei diese Prozentzahl die Menge an pyroGlu-MCP-1 (ermittelt z.B. als Fläche unter der Kurve in einem HPLC-Elutionsprofil) bezogen auf die in der Lösung vorhandene Menge an Gesamt-MCP-1 (also einschließlich eines ggf. noch verbliebenen Gln-MCP-1-Anteils und möglicher Oxidations- und anderer Neben- oder Abbauprodukte) angibt.

Gemäß einer weiteren Ausführungsform der Erfindung wird ein Verfahren zur Herstellung einer pyroGlu-MCP-1-Präparation bereitgestellt, das zumindest die folgenden Schritte beinhaltet:

- Herstellen einer Gln-MCP-1-Präparation auf rekombinantem Wege durch Expression eines für MCP-1 kodierenden Genkonstrukts in einer Wirtszelle,
- optional Aufkonzentrieren und/oder Aufreinigen des Gln-MCP-1, das in der Gln-MCP-1-Präparation enthalten ist, und schließlich
- Konvertieren des Gln-MCP-1, das in der Gln-MCP-1-Präparation enthalten ist bzw. vor der Aufkonzentrierung und/oder dem Aufreinigen enthalten war, zu einer pyroGlu-MCP-1-Präparation, die die Proteinmolekülspezies pyroGlu-MCP-1 enthält, wobei dieser Schritt gemäß dem obenstehend beschriebenen "Verfahren zur Herstellung von pyroGlu-MCP-1 aus rekombinant hergestelltem Gln-MCP-1" ausgeführt wird.

Optional kann dieses Verfahren außerdem z.B. noch das Umpuffern der pyroGlu-MCP-1-Präparation oder die weitere Aufreinigung beinhalten, so z.B. durch einen anschließenden Säulenchromatographie-Schritt, eine Dialyse, eine Ultrafiltration, etc.

Verfahren zur biotechnologischen Herstellung rekombinanter Proteine sind bekannt. Sie umfassen insbesondere Fermentations-, Reinigungs-, Konzentrations-, und andere Schritte. Der oben erläuterte Konvertierungsschritt kann innerhalb eines solchen "Mehrschrittverfahrens" an verschiedenen Positionen eingefügt werden, also z.B. mit einer noch weitgehend ungereinigten oder auch einer bereits voll aufgereinigten MCP-1-Proteinlösung als Ausgangslösung. Insbesondere denkbar ist auch ein Verfahrensablauf, bei dem zwischendurch noch nicht (vollständig) konvertiertes Gln-MCP-1 in mehr, weniger oder praktisch vollständig gereinigter Form zwecks Verbesserung der Lagerfähigkeit in noch unkonvertierter Form lyophilisiert wird und zu gegebener Zeit wieder in Lösung gebracht und dann gemäß dem obigen Verfahren in pyroGlu-MCP-1 überführt wird. Natürlich kann auch nach der Konvertierung zu pyroGlu-MCP-1 die erhaltene Proteinlösung lyophilisiert werden.

Das Verfahrensprodukt, nämlich die der N-terminalen Modifizierung (Konvertierung) unterzogene pyroGlu-MCP-1-Präparation, enthält erfindungsgemäß bezogen auf den Gesamtgehalt an MCP-1 (konvertiertes plus nicht-konvertiertes Protein plus z.B. durch Oxidation entstandene Nebenprodukte) einen Anteil an pyroGlu-MCP-1 von mindestens 90 %, vorzugsweise mindestens 95 % und weiter vorzugsweise mindestens 96 %.

Mit der Bezeichnung (Gln- bzw. pyroGlu-MCP-1-)"Präparation" soll ausgedrückt werden, dass in den verschiedenen Verfahrensstufen das MCP-1-Protein in gelöster Form in einer (Puffer-)Lösung vorliegt und damit neben dem MCP-1 noch weitere Komponenten vorhanden sein können, so jedenfalls die Ionen des verwendeten Puffersalzes und dazu ggf. weitere Salze, Antioxidanzien, Stabilisatoren, antimikrobielle Reagenzien, Detergenzien, Konservierungsmittel, Komplexbildner, etc.

Gemäß einem weiteren Aspekt der Erfindung wird eine Zusammensetzung bereitgestellt, die pyroGlu-MCP-1 oder eine pyroGlu-MCP-1-Präparation enthält, die nach den zuvor genannten Verfahren hergestellt wurde, wobei mindestens 90 % des in der Zusammensetzung enthaltenen MCP-1 (konvertiertes plus nicht-konvertiertes Protein plus Nebenprodukte; also die "Fläche unter der Kurve" z.B. in einem HPLC-Elutionschromatogramm) in Form des pyroGlu-MCP-1 vorliegen.

Gegenstand der Erfindung ist damit außerdem eine auf rekombinantem Weg insbesondere in Prokaryonten und besonders bevorzugt in *E. coli* hergestellte MCP-1-Präparation, in der zumindest 90 % des MCP-1-Proteins als pyroGlu-MCP-1 vorliegen. Bei der Herstellung in gängigen Expressionszellen wird häufig das Protein - anders als bei nativer Produktion - nicht das natürliche Glykosylierungsmuster zeigen. Insbesondere bei Herstellung in Prokaryonten wie z.B. *E. coli* wird das erhaltene pyroGlu-MCP-1 bzw. die erhaltene pyroGlu-MCP-1-Präparation im Gegensatz zu der bei nativer Expression im menschlichen Organismus vorkommenden Form nicht glykosyliert und/oder sialylyliert sein und sich so von einem solchen nativen Protein bzw. einer aus nativer Quelle erhaltenen Proteinpräparation unterscheiden.

Eine derartige erfindungsgemäße Zusammensetzung kann insbesondere ein Arzneimittel oder eine pharmazeutische Zusammensetzung sein, die, neben einem zu mindestens 90 %, 95% oder 96% betragenden Anteil von pyroGlu-MCP-1 am Gesamt-MCP-1-Gehalt (konvertiertes plus nicht-konvertiertes Protein plus Nebenprodukte) noch übliche Hilfsund Trägerstoffe, Salze, Antioxidanzien, Stabilisatoren, antimikrobielle Reagenzien, Detergenzien, Konservierungsmittel, Komplexbildner, etc. enthält.

Überraschenderweise wurde also gefunden, dass - anders als erwartet - die Inkubation einer auf rekombinantem Wege hergestellten MCP-1-Proteinpräparation in wässriger Lösung und bei erhöhter Temperatur nicht zu deren Abbau und biologischer Inaktivierung führt, sondern über diesen aus biotechnologischer Sicht sehr ungewöhnlichen und an sich in der Regel unerwünschten Verfahrensschritt - bei geeigneter Wahl einer Mehrzahl von Parametern - eine überraschend homogene, biologisch voll aktive pyroGlu-MCP-1-Präparation erhalten werden kann, die darüber hinaus dann ihrerseits auch bei längerer Lagerung ihre Homogenität beibehält, also stabiler als die Gln-MCP-1-(Ausgangs-) Präparation ist.

Behörden wie die Food and Drug Administration in den USA oder die EMEA in Europa machen die Erteilung einer arzneimittelrechtliche Marktzulassung eines Pharmazeutikums von der Erfüllung zahlreicher, letzlich den Patienten schützenden, den Arzneimittelherstellern auferlegten Bedingungen abhängig. So müssen von dem jeweiligen Hersteller Belege vorgelegt werden, die z.B. die Reinheit des pharmazeutischen Produktes, dessen Lagerstabilität und dessen reproduzierbare Herstellbarkeit zeigen. Diese drei genannten Aspekte sind nur eine kleine Auswahl, spielen aber gerade bei der Zulassung von Biopharmazeutika, also Arzneimitteln, die makromolekulare, aus Naturstoffen bestehende oder von diesen abgeleitete Wirkstoffe (Proteine, Nukleinsäuren, Proteoglycane, Polysaccharide, etc.) enthalten, eine zentrale Rolle. Häufig sind die im Rahmen dieser Zulassung gestellten Anforderungen an das (Bio-) Pharmazeutikum sehr schwer zu erfüllen. Z.B. Wirkstoffe beruhend auf einem Protein müssen nach ihrer in einem mehr oder weniger komplexen zellulären Organismus erfolgten Produktion einer intensiven und komplizierten, mehrstufigen Reinigung unterzogen werden und dürfen dabei nicht - z.B. durch Oxidationen oder andere spontane chemische Reaktionen - in unkontrollierter Weise in ihrer Struktur verändert werden. Darüber hinaus sollte das Endprodukt eine akzeptable Lagerstabilität besitzen um nicht komplizierte und teure Versorgungsnetze zwischen Hersteller und anwendendem Arzt oder Patient oder anwendender Klinik bereitstellen zu müssen - ein Erfordernis, das insbesondere bei Proteinen, bei denen es darauf ankommt, die korrekte Sekundärstruktur (Faltung) aufrechtzuerhalten, in der Regel nur unter großen Anstrengungen zu erfüllen ist.

Im Falle des MCP-1-Proteins reicht die Qualität kommerziell erhältlicher Präparationen für z.B. *in vitro*-Versuche in vielerlei Hinsicht häufig aus. Wie die Erfinder jedoch im Zuge ihrer Arbeiten zu Tage gefördert haben, eignen sich selbige Präparationen gar nicht gut um ein zulassungsfähiges Pharmazeutikum herzustellen. Bereits ein kurzes Stehenlassen in Lösung bei Raumtemperatur vor Verabreichung am Patienten, wie es z.B. in Arztpraxen oder Krankenhäusern nie ganz ausgeschlossen werden kann, führt nach Erkenntnissen der Erfinder zum Auftreten ursprünglich als Verunreinigung angesehener "Abbau-"Produkte in den MCP-1-Präparationen. Eine verstärkte Aufreinigung des ursprünglich als Wirkstoff vorgesehenen Gln-MCP-1 konnte kurzfristig einen hohen Reinheitsgrad wiederherstellen, auch diese Präparation war aber erneut gegenüber einer kurzzeitigen Aufbewahrung in Lösung und bei Raumtemperatur nicht stabil. Das Betreiben eines arzneimittelrechtlichen Zulassungsverfahrens auf der Basis einer solch instabilen Wirkstoffpräparation ist mit enormen Schwierigkeiten verbunden, wenn nicht schlichtweg aussichtslos.

Überraschend konnten gemäß der Lehre der Erfindung diese Probleme überwunden werden, indem ein ungewöhnlicher, auf den ersten Blick hochgradig kontraproduktiver Verfahrensschritt, nämlich die Inkubation bei erhöhter Temperatur (die ja nach früherer Anschauung gerade die Inhomogenität förderte) in das eigentliche Herstellungs- und Aufreinigungsverfahren eingeschlossen wurde. Durchgeführt unter von den Erfindern im Detail analysierten und optimierten Bedingungen kann eine solche Inkubation nämlich eine fast quantitative Konvertierung des ursprünglichen eigentlichen Wirkstoffs Gln-MCP-1 in die ursprünglich als Verunreinigung angesehene pyroGlu-MCP-1-Form bewirken. Letztere Form ist dann gegenüber proteindenaturierenden Einflüssen wie Inkubation bei erhöhter Temperatur o.ä. erstaunlich stabil. Somit konnte eine MCP-1-Präparation bereitgestellt werden, die sich aufgrund hoher Reinheit und Homogenität, hervorragend reproduzierbarer Herstellbarkeit und ihrer Lagerstabilität für die Verwendung als Arzneimittelwirkstoff eignet, die gute Chancen besitzt, in entsprechenden Zulassungsverfahren die genannten strengen Anforderungen zu erfüllen, und die damit neue, auf der Wirkung von MCP-1 beruhende therapeutische Verfahren ermöglicht.

Gemäß einem Teilaspekt der Erfindung wird eine nach dem oben und in den Ansprüchen genannten Verfahren hergestellte pyroGlu-MCP-1-Präparation

- zur therapeutischen Behandlung von Gefäßverschlusserkrankungen wie insbesondere von Koronararterienerkrankungen (coronary artery disease, CAD), peripheren Arterienverschlusserkrankungen (peripheral arterial occlusive disease, PAOD), zerebralen und mesenterialen arteriellen Verschlusserkrankheiten, oder
- zur Herstellung eines Arzneimittels zur Behandlung von Gefäßverschlusserkrankungen wie insbesondere von Koronararterienerkrankungen (coronary artery disease, CAD), peripheren Arterienverschlusserkrankungen (peripheral arterial occlusive disease, PAOD), zerebralen und mesenterialen arteriellen Verschlusserkrankheiten verwendet.

Die Erfindung ermöglicht auch die Durchführung eines Verfahrens zur Behandlung eines an den genannten Gefäßverschlusserkrankungen leidenden Patienten, wobei die durch Konvertierung hergestellte pyroGlu-MCP-1-Präparation oder das unter Verwendung einer solchen pyroGlu-MCP-1-Präparation hergestellte Arzneimittel verabreicht wird, z.B. durch Injektion oder Infusion.

Bezüglich der einzelnen optimierten Verfahrensparameter ist noch zu bemerken, dass es den Biotechnologen nicht nur einiges an Überwindung kostet, eine Proteinpräparation über einen längeren Zeitraum von u.U. mehreren Tagen bei erhöhten Temperaturen von z.B. auch 60 °C zu inkubieren. Bezüglich des oben als optimal offenbarten, neutralen bis sauren pH-Werts der Inkubationslösung steht dem eigentlich die chemische Logik entgegen: Die Konvertierung von Glutamin zum zyklischen Pyroglutamat ist eine nukleophile Substitutionsreaktion, bei der ein freies Elektronenpaar am angreifenden Stickstoffatom der C-alpha-Aminogruppe des Glutamats am C-Atom der C-gamma-Amidgruppe angreift. Ein saurer pH-Wert würde an sich zu einer verstärkten Protonierung der C-alpha-Aminogruppe führen und sich daher negativ auf die Reaktionsgeschwindigkeit auswirken. Dies ist nach Erkenntnissen der Erfinder aber gerade nicht der Fall. Einenicht einschränkend zu interpretierende - denkbare Erklärung könnte sein, dass das gefaltete MCP-1-Protein für den N-Terminus eine Mikroumgebung bildet, in der trotz saurem pH-Werts der umgebenden wässrigen Lösung die Aminogruppe unprotoniert vorliegt, also ein freies Elektronenpaar für den nukleophilen Angriff zur Verfügung steht.

## Beispiele

Beispiel 1: Herstellung einer pyroGlu-MCP-1-Präparation gemäß dem erfindungsgemäßen Verfahren

Fermentation:

Die Fermentation wurde mit einem Stamm von *Escherichia coli* K12 (W3110), durchgeführt. Der Stamm wurde mit einem ColE1-Plasmid (pBR322-Derivat) transformiert, welches folgende Elemente enthält: die genomische Sequenz von hMCP-1 unter der Kontrolle eines allgemein gebräuchlichen Promoters (Phosphatase, pPhoA), ein ColE 1-Replikationsursprung und das Resistenzgen für Tetrazyklin. Zur Fermentation wurde der Produktionsstamm im Schüttelkolben in LB Medium, das Tetracyclin enthielt, vorkultiviert. Die Inkubation erfolgte bei 37°C bis eine OD von 1 erreicht war. Die Vorkultur wurde in den Fermenter transferiert und unter Rühren und Luftzufuhr bei 37°C weitergezüchtet. Das Medium enthielt Glukose, diverse Salze, Spurenelemente, Hefeextrakt, Aminosäuren und Tetracyclin. Der pH-Wert wurde mit Ammoniak auf 6,8 gehalten. Sobald die vorgelegte Glukose verbraucht war, wurde durch Zufuhr von Glukose der Gelöstsauerstoff ($PO_2$) auf dem Sollwert von 40% konstant gehalten. Die Induktion des Phosphatasepromotors erfolgte automatisch, sobald das Phosphat im Medium verbraucht war. Nach 39 Stunden wurde die Biomasse mit einer Röhrenzentrifuge (CEPA) geerntet und bei -70°C gelagert.

Zellaufschluss und Proteinreinigungsschritte:

Das gefrorene Zellpellet wurde in der vierfachen Menge Lysepuffer (200 mM Tris, 55 mM NaCl, 5mM EDTA, pH 7,5) mit einem Ultraturrax resuspendiert. Der Zellaufschluss erfolgte durch zwei Passagen mit einem Homogenisator bei 460 bar. Zellbruchstücke wurden mit einer CEPA Zentrifuge entfernt. Der Überstand wurde optional mit Polysep II (1,2 µm) - Filtern (Millipore) filtriert, und dann auf eine Säulenkombination bestehend aus einer Q Sepharose FF und einer SP Sepharose FF geladen. Die Säulen waren mit Lysepuffer equilibriert.

Nach Ende des Beladens wurde die Säulenkombination mit Lysepuffer gewaschen und die Q Sepharose - Säule abgeklemmt. Die SP Sepharose wurde weiter mit Lysepuffer, anschließend mit 5 M Harnstoff (in Lysepuffer) und wieder mit Lysepuffer gewaschen. Die Elution des Produkts erfolgte in einem linearen NaCl Gradienten.

Das Eluat wurde mit Ammoniumsulfat bis zu einer Endkonzentration von 1,3 mol/L aufgesalzen und 15 min bei 3200 g zentrifugiert. Der Überstand wurde auf eine Phenyl Sepharose-Säule geladen, die mit 1,3 M Ammoniumsulfat (in Lysepuffer) equilibriert war.

Der Durchfluss der Phenyl-Sepharose wurde auf eine Source 30 RPC-Säule aufgetragen, die mit Lysepuffer equilibriert war. Das Nachwaschen erfolgte mit Lysepuffer, Wasser und Puffer A (5% EtOH, 0,1% TFA). Das Produkt wurde in einem linearen Gradienten von 20% Puffer B (95% EtOH, 0,1% TFA) auf 70% Puffer B in 10 Säulenvolumina eluiert.

Konvertierungsschritt:

Das Eluat wurde in unterschiedlichen Puffern, z.B. Phosphatpuffer, 10 - 40 mM, pH 6,0 - 7,4 bis zu 1:10 verdünnt. Als End - pH der Konvertierungslösung wurde gemäß einer bevorzugten Ausführungsform ein Wert zwischen 6,0 und 6,5 angestrebt. Die Proteinkonzentration lag zwischen 0,2 und 1,0 mg/mL.
Die Lösung wurde sterilfiltriert und unter leichtem Schütteln (60 rpm) bei Temperaturen von (in unterschiedlichen Ansätzen) 35 - 80°C inkubiert. Der Reaktionsverlauf wurde durch HPLC-Analysen verfolgt.

Endreinigungsschritte:

Sobald das Reaktionsende erreicht war, wurde die Konvertierungslösung auf eine SP Sepharose HP - Säule geladen, die in Puffer A (40 mM Phosphat, pH 6,0) equilibriert war. Die Elution erfolgte mit einem linearen NaCl Gradienten. Die NaCl - Konzentration im Eluat war etwa 350 mmol/L, die Proteinkonzentration etwa 3 mg/mL. Das Eluat wurde mit Puffer A auf eine Leitfähigkeit verdünnt, die 250 mM NaCl entspricht. Anschließend wurde mit 40 mM Phosphat, pH 6,0, 250 mM NaCl weiterverdünnt bis die Proteinkonzentration 1 mg/mL betrug. Die so entstandene Bulklösung wurde sterilfiltriert und bei 4°C oder -70°C bis zur Formulierung gelagert.

Analytik:

Der Verlauf des oben erläuterten Konvertierungsschritts wurde per RP-HPLC verfolgt. Chromatogramme der Elutionsprofile wurden vor Start der Konvertierungsreaktion ($t_0$) und zu den Zeitpunkten t = 24 h, t= 48 h und t = 120 h aufgenommen. Wie aus den in Fig. 1 gezeigten vier Chromatogrammen zu erkennen ist, waren bei einer Inkubationstemperatur von 35 °C nach 48 h ca. 90 % des Proteins zu pyroGlu-MCP-1 konvertiert.

Fig. 2 zeigt die Kinetik der Umsetzung von Gln-MCP-1 zu pyroGlu-MCP-1 unter den beschriebenen Bedingungen. Darin ist zu erkennen, dass bei zunehmender Reaktionsdauer eine in Fig. 2 als "Variant 2" bezeichnete, nicht näher zu charakterisierende Verunreinigung in Erscheinung tritt.

Ähnliche Reaktionsverläufe wurden bei Durchführung des Konvertierungsschrittes in 0.1 M Natriumacetatpuffer, pH 5.5, oder 0.1 M Natriumcitratpuffer, pH 3.5, beobachtet (Daten nicht gezeigt).

Die zuvor beschriebenene Reaktion wurde wiederholt, außer dass anstelle einer Reaktionstemperatur von 35 °C Temperaturen von 24 °C, 60 °C und 80 °C gewählt wurden. Wie anhand der in Tabelle 3 zusammengefassten Daten ersichtlich ist, waren bei 24 °C in vertretbarer Zeit keine zufriedenstellenden Ausbeuten an pyroGlu-MCP-1 zu erhalten. Bei 60 °C und 80 °C waren dagegen signifikant höhere Reaktionsgeschwindigkeiten zu beobachten. Die Reaktionsrate bei 80 °C betrug in etwa das 50fache der Rate bei Raumtemperatur.

Tabelle 3:

| | Temperatur [°C] | Geschw.konst. [1/h] | Halbwertzeit [h] | Dauer | Reinheit [%] |
|---|---|---|---|---|---|
| Vergleichsversuch 1: 20 mM Phosphat pH 6,0 | 24 | 0,031 | 22,4 | 7,5 d | n.d. |
| 20 mM Phosphat pH 6,0 | 35 | 0,089 | 7,8 | 2,6 d | 96,6 |
| 20 mM Phosphat pH 6,0 | 60 | 0,660 | 1,1 | 8,8 h | 95,0 |
| 20 mM Phosphat pH 6,0 | 80 | 1,670 | 0,4 | 3,2 h | 91,3 |
| 20 mM Phosphat pH 6,0 150 mM NaCl | 35 | 0,041 | 16,9 | 5,6 d | n.d. |
| 20 mM Phosphat pH 6,0 5% EtOH | 35 | 0,108 | 6,4 | 2,1d | 95 |
| Vergleichsversuch 2: 20 mM Phosphat pH 8,5 | 35 | 0,037 | 18,7 | 6,2 d | n.d. |
| PBS Tween, 3,5% EtOH, 0,01% TFA pH 7,4 | 35 | 0,040 | 17,3 | 5,7 d | 95 |
| n.d. = not determined, nach der angegebenen Dauer war das Reaktionsende noch nicht erreicht | | | | | |

Eine höhere Reaktionstemperatur begünstigt allerdings auch die Bildung von Neben- oder Abbauprodukten, was den Reinheitsgrad - insbesondere im Falle der Durchführung des Versuchs bei 80°C - reduziert (Tabelle 3).

In Fig. 3 sind in hoher Auflösung die Chromatogramme des Elutionsprofils von pyroGlu-MCP-1-Präparationen gezeigt, die unter den in der Tabelle 3 zusammengefassten Bedingungen (20 mM Phosphatpuffer, 35 °C, 60 °C bzw. 80 °C) erhalten wurden. Wie hieraus zu erkennen ist, treten bei hohen Temperaturen selbst bei kurzer Inkubationsdauer (z.B. 3 Stunden bei 80 °C) deutlich mehr Verunreinigungen mit MCP-1-Neben- oder - Abbauprodukten auf als bei einer fünftägigen Inkubation bei 35 °C.

Der bei bestimmten Verfahrensausführungen produktionstechnisch bedingte Gehalt an (z.B. 5 %) Ethanol (EtOH) wirkte sich bei ansonsten konstanten Versuchsbedingungen (20 mM Phosphatpuffer, pH 6.0) nicht negativ auf die Reaktionsraten oder den Reinheitsgrad des Produkts aus.

Beispiel 2: Herstellung einer pyroGlu-MCP-1-Präparation gemäß einem alternativen erfindungsgemäßen Verfahren

Die Fermentation, der Zellaufschluss und die Proteinreinigungsschritte wurden wie in Beispiel 1 erläutert durchgeführt.

Konvertierungsschritt:

Das Eluat der Source 30 RPC wurde 1:10 mit PBS, 0,02% Tween 20 verdünnt. Die Proteinkonzentration betrug dann etwa zwischen 0,2 und 0,5 mg/mL, der pH etwa 7,4. Die Lösung enthielt außer den Pufferkomponenten etwa 3,5% EtOH und 0,01% TFA, die aus dem Eluat des vorangegangenen Source 30 RPC -Schrittes stammten. Die Lösung wurde mit einem Millipak 20 (0,2 µm, Millipore) steril in Polypropylenflaschen filtriert und unter leichtem Schütteln bei 35°C inkubiert. Der Reaktionsverlauf wurde durch HPLC-Analysen verfolgt. Sobald das Reaktionsende erreicht war, wurde die Konvertierungslösung mit einer Pellikon 2 UDF-Membran (3k, Millipore) gegen PBS, 0,02% Tween 20 ultradiafiltriert und die Proteinkonzentration auf 0,1 mg/mL eingestellt. Die Abfüllung erfolgte steril über einen Millipore GV-Filter (0,22µm) in 1mL-Portionen in Glasgefäße.

Analytik:

Der Verlauf des Konvertierungsschritts wurde per RP-HPLC verfolgt. Wie in Fig. 4 zu sehen ist, waren nach 5 Tagen mehr als 90 % des Proteins zu pyroGlu-MCP-1 konvertiert, wobei überraschenderweise trotz der langen Inkubationsdauer ein sehr hoher Reinheitsgrad von 95 % erhalten wurde (Tabelle 3). Wie aus einem Vergleich zwischen Ansätzen mit 20 mM Phosphatpuffer und solchen mit 20 mM Phosphatpuffer plus 150 mM Salz (Beispiel 1, Tabelle 3, Zeile 3 vs. Zeile 6) zu entnehmen ist, dürfte die relativ niedrige Geschwindigkeitskonstante in Ansätzen mit PBS als Inkubationslösung zurückzuführen zu sein auf die höhere Salzkonzentration, Ionenstärke oder Osmolarität.

Beispiel 3: Herstellung eines Arzneimittels auf der Basis der nach Beispiel 1 oder Beispiel 2 hergestellten MCP-1-Präparation

Die in Beispiel 1 oder Beispiel 2 erhaltene pyroGlu-MCP-1-Präparation wurde in PBS (Natriumchlorid, Dinatriumhydrogenphosphat, Kaliumchlorid, Kaliumdihydrogenphosphat; pH 7.0), enthaltend außerdem 0,02 % Tween 20, auf eine Konzentration von 0,1 mg/mL verdünnt und in 1-mL-Volumina in Glasgefäße abgefüllt. Die fertige Arzneimittellösung war klar, farblos und geruchlos und kann per Injektion oder Infusion verabreicht werden.

Beispiel 4: Vergleich der biologischen Aktivität von pyroGlu-MCP-1 und Gln-MCP-1

Messprinzip:

MCP-1 bindet an und aktiviert den MCP-1 Rezeptor CCR2b. Die Rezeptoraktivierung führt zu einem Calciumeinstrom ins Cytosol. Dieser kann mit einem Fluorescence Imaging Plate Reader (FLIPR; Molecular Devices) gemessen werden. Hierzu wird in Zellen, die hCCR2b an ihrer Oberfläche exprimieren, der inaktive Fluoreszenzfarbstoffester Fluo-4 AM eingeschleust, der dann durch intrazelluläre Esterasen gespalten wird. In dieser Form bindet der Fluoreszenzfarbstoff $Ca^{2+}$-Ionen. Bei einer Anregung mit einer Wellenlänge von 488 nm kommt es zu einer Emission mit einem Maximum bei 528 nm. Die Emissionsintensität ist abhängig von der cytosolischen Calciumkonzentration. Die Intensitätsänderung des emittierten Lichts korreliert also mit der cytosolischen Calciumkonzentration welche wiederum vom Aktivierungszustand des Rezeptors abhängig ist.

Ein charakteristisches zeitaufgelöstes Messsignal nach Zugabe von MCP-1 ist in Fig. 5 gezeigt. Hieraus lässt sich erkennen, dass es nach der Zugabe von MCP-1 zu einer schnellen Calcium-Freisetzung kommt, die mit einem starken Anstieg der Fluoreszenz verbunden ist. Das Maximum (b) wird dabei bereits wenige Sekunden nach der Applikation (a) erreicht. Der Zeitraum zwischen Applikation (a) und maximaler Stimulation (b) beträgt ungefähr 20-30 sec.

In den nachfolgend beschriebenen Versuchen wurde die Auswertung anhand der Maximalwerte vorgenommen, da diese von Sekundäreffekten, wie z. B. einem Calciuminduzierten Calciumeinstrom, weniger beeinflusst werden und damit eine exaktere Messung ermöglicht wird.

Bereitstellung stabil exprimierender CHO/hCCR2B-K1 Zellen:

Die kodierende Region des humanen CCR2b Rezeptors (Genbank-Accession No: D29984) wurde über PCR amplifiziert. Anschließend wurde das 1.08 kb BamHI-XbaI Fragment in einen Expressionsvektor einkloniert. Mit diesem als Expressionsvektor dienenden Plasmid wurden CHO-K1-Zellen transfiziert.

Die Zellen wurden in einem auf Ham's F12 Medium basierenden Kulturmedium kultiviert und regelmäßig passagiert. Am Vortag der Messung wurden 5000 Zellen in 384-well-Assayplatten (Corning Costar) mit 40 µl Kultivierungsmedium ausplattiert und über Nacht (ca. 24 h) bei 37 °C, 5 % $CO_2$, 95 % relativer Feuchte anhaften gelassen. In allen Assays wurden vierfach-Bestimmungen durchgeführt.

Am Versuchstag wurden zunächst die Substanzplatten hergestellt. Als Verdünnungspuffer für die verschiedenen MCP-1-Präparationen wurde Hanks Puffer mit 0.1 % BSA (proteasefrei) verwendet. Als Leerkontrolle diente Hanks Puffer mit 0.1 % BSA. Anschließend wurden zu den Zellen 40 µl / well Fluo-4 Färbemedium zugegeben und die Ansätze für 45 min. bei 37 °C, 5 % $CO_2$, 95 % relativer Feuchte inkubiert. Danach wurden die gefärbten Zellen viermal mit 60 µl Waschpuffer gewaschen, wobei ein Rest von 25 µl des Waschpuffers pro well verblieb. Die Zellen wurden danach weitere 5 min bei Raumtemperatur mit Waschpuffer inkubiert.

Zur Fluoreszenzmessung wurde das FLIPR Meßgerät so eingestellt, dass sich gefärbte und ungefärbte wells mindestens um einen Faktor 1:5 unterschieden und die gefärbten wells ca. 11.000 fluorescence counts aufwiesen. Die weiteren FLIPR-Einstellungen waren:

| | |
|---|---|
| Excitation | 488 nm |
| Emission | 510 - 570 nm (bandpass) |
| Negative correction | Hanks/BSA (= Leerkontrollenabgleich) |
| Bias substraction | 6 (= Nivellierung aller wells vor Substanzzugabe auf 0) |
| Presoak der tips | mit 25 µl der jeweiligen Substanzlösung aus der Substanzplatte (= Minimierung eines Adhäsionsartefakts) |

Der Meßverlauf bestand aus folgenden Schritten:

6 Intervalle im 5 sec. Takt

15 µl Substanzzugabe
60 Intervalle im 1 sec. Takt
18 Intervalle im 5 sec Takt

Zur Auswertung wurde das Maximalsignal der 78 Intervalle nach Substanzzugabe verwendet. Als Kontrollansätze dienten:

| | |
|---|---|
| Leerkontrolle | zur negative correction |
| Positivkontrolle (ATP) | zur Überprüfung der Färbung |
| Referenzkontrolle („Standard") | -Kontrolle der Rezeptorexpression auf den Zellen<br>- Berechnungsgrundlage für die Aktivitätsbestimmung unbekannter Proben |

Die zu testenden Proben wurden parallel mit der Referenzkontrolle in identischen Verdünnungsschritten pipettiert. Die in die Versuche eingesetzten Konzentrationen wurden so gewählt, dass sie im $EC_{50}$-Bereich der Referenzkontrolle lagen.

Vergleich erfindungsgemäßer und nicht-erfindungsgemäßer MCP-1-Präparationen:

Es wurden zwei nach dem erfindungsgemäßen Verfahren hergestellte pyroGlu-MCP-1-Präparationen ("Batch 071100kh" und "Batch 0141921") und eine auf rekombinantem Weg hergestellte MCP-1-Präparation, deren N-terminaler Glutamin-Rest nicht in dem oben beschriebenen, erfindungsgemäßen Verfahrensschritt zu einem pyroGlutamat-Rest konvertiert worden war (MCP-1-Präparationen erhältlich von Peprotech) im zuvor erläuterten Versuchssystem getestet und verglichen. Aus den wie beschrieben aufgenommenen Messkurven wurde der $EC_{50}$-Wert ermittelt (s. Fig. 6).

Für die beiden pyroGlu-MCP-1-Präparationen ergaben sich praktisch identische $EC_{50}$-Werte, während die Werte für die nicht-erfindungsgemäße, nicht N-terminal modifizierte MCP-1-Präparation hiervon deutlich abwichen (Fig. 6). Der $EC_{50}$-Wert letzterer Präparation erwies sich als um einen Faktor 2 bis 3 schlechter (pyroGlu-MCP-1: $EC_{50}$ = 7,82 nM; MCP-1-Präparation von Peprotech: $EC_{50}$ = 20,76 nM).

Die biologische Aktivität der beiden Präparationen wurde hieraus wie folgt berechnet:

Zuerst wird eine positive correction auf das pyroGlu MCP-1 bei der Konzentration le-8 M durchgeführt, d.h. der bei einer Konzentration von 10 nM pyroGlu-MCP-1 erzielte Wert wurde auf 100 % gesetzt. Dies ist im fast linearen Bereich des Kurvenanstiegs. Anhand dieses prozentualen Signals wird dann die Aktivität anderer Präparationen, wie z.B. des MCP-1 von Peprotech nach der Formel

$$\%\ \text{Aktivität}_{(\text{Probe})} = \text{RFU}_{(\text{Probe bei 1e-8M})} \times 100\ \%\ /\ \text{RFU}_{(\text{Standard bei 1e-8M})}$$

berechnet. Hieraus ergibt sich im obigen Beispiel, wie in Tabelle 4 gezeigt, ein Wert von 48 % Aktivität für die nicht-N-terminal-modifizierte MCP-1-Präparation bezogen auf die pyroGlu-MCP-1-Präparation.

Tabelle 4:

Meßwerte: (neg.corr.:Hanks/BSA; bias substraction:6)

| | Wert 1 | Wert 2 | Wert 3 | Wert 4 |
|---|---|---|---|---|
| pyroGlu MCP-1 (071100kh) bei 1e-8M | 12137,63 | 13919,36 | 12286,6 | 13249,68 |
| MCP-1 (Peprotech) bei 1e-8M | 6216,81 | 6125,89 | 6836,11 | 5425,31 |

| | Mittelwert | SD |
|---|---|---|
| pyroGlu MCP-1 (071100kh) bei 1e-8M | 12898,3 | 727,8 |
| MCP-1 (Peprotech) bei 1e-8M | 6151,0 | 500,2 |

$\%Aktivität_{(MCP\text{-}1\ (Peprotech))}$ = 6151 x 100% / 12898.3 = 48 % Aktivität

Beispiel 5: Temperatursensitivität von pyroGlu-MCP1

PyroGlu-MCP-1 wurde 1 bzw. 2 Stunden bei 56°C oder 95°C inkubiert. Die danach noch vorhandene biologische Aktivität wurde dann anhand des Testsystems des Beispiels 4 gemessen.

Es zeigte sich, dass sowohl eine einstündige als auch eine zweistündige Inkubation einer pyroGlu-MCP-1-Präparation bei 56 °C keine Denaturierung verursacht. Dagegen führte, wie in Fig. 7 gezeigt ist, die Inkubation bei 95 °C zu einem sehr deutlichen Denaturierungseffekt.

**Patentansprüche**

1. Verfahren zur Herstellung von pyroGlu-MCP-1 aus rekombinant hergestelltem Gln-MCP-1, bei dem Gln-MCP-1

   - bei einer Temperatur im Bereich von 30°C und 80°C
   - in einer Pufferlösung mit

     -- einer Salzkonzentration im Bereich von 10 mM bis 160 mM und
     -- einem pH-Wert im Bereich von 2 bis 7.5

   inkubiert wird bis mindestens 90 % des MCP-1 in Form des pyroGlu-MCP-1 vorliegen.

2. Verfahren nach Anspruch 1, bei dem die Pufferlösung ein Phosphatpuffer mit einer Konzentration im Bereich von 20 mM bis 50 mM und mit einem pH-Wert im Bereich von 3.5 bis 6.5 ist.

3. Verfahren nach einem der Ansprüche 1 oder 2, bei dem die Pufferlösung zusätzlich ein Detergens, ein Antioxidationsmittel, ein Konservierungsmittel, einen Stabilisator, ein antimikrobielles Reagens und/oder einen Komplexbildner enthält.

4. Verfahren zur Herstellung einer pyroGlu-MCP-1-Präparation, beinhaltend zumindest die Schritte

   - Herstellen einer Gln-MCP-1-Präparation durch Expression eines für MCP-1 kodierenden Genkonstrukts in einer Wirtszelle,
   - optional Aufkonzentrieren und/oder Aufreinigen des Gln-MCP-1 der Gln-MCP-1-Präparation,
   - Konvertieren des Gln-MCP-1 der Gln-MCP-1-Präparation zu einer pyroGlu-MCP-1 einhaltenden pyroGlu-MCP-1-Präparation nach einem der Verfahren 1 bis 3, und
   - optional Umpuffern und/oder weitere Aufreinigung der pyroGlu-MCP-1-Präparation,

   wobei der Anteil an pyroGlu-MCP-1 bezogen auf den Gesamtgehalt an MCP-1 in der resultierenden pyroGlu-MCP-1-Präparation mindestens 90 % beträgt.

**5.** Zusammensetzung enthaltend eine pyroGlu-MCP-1-Präparation hergestellt nach Anspruch 4, wobei mindestens 90 % des in der pyroGlu-MCP-1-Präparation enthaltenen MCP-1 in Form des pyroGlu-MCP-1 vorliegt.

**6.** Verfahren zur Herstellung eines pyroGlu-MCP-1 enthaltenden Arzneimittels, bei dem eine nach Anspruch 4 hergestellte pyroGlu-MCP-1-Präparation verwendet wird.

**7.** Arzneimittel oder pharmazeutische Zusammensetzung enthaltend eine pyroGlu-MCP-1-Präparation hergestellt nach Anspruch 4, wobei mindestens 90 % des darin enthaltenen MCP-1 in Form des pyroGlu-MCP-1 vorliegt.

**8.** Arzneimittel oder pharmazeutische Zusammensetzung nach Anspruch 7, enthaltend pyroGlu-MCP-1 in einem Phosphatpuffer mit Natriumchlorid und optional einem Detergens als Zusatzstoff.

**9.** Verwendung von pyroGlu-MCP-1 oder einer nach Anspruch 4 hergestellten pyroGlu-MCP-1-Präparation zur Herstellung eines Arzneimittels zur Behandlung von Gefäßverschlusserkrankungen wie insbesondere von Koronararterienerkrankungen (coronary artery disease, CAD), peripheren Arterienverschlusserkrankungen (peripheral arterial occlusive disease, PAOD), zerebralen und mesenterialen arteriellen Verschlusserkrankheiten.

**10.** Nach Verfahren 4 hergestellte rekombinante MCP-1-Präparation, deren biologische Aktivität bezüglich einer rekombinant hergestellten MCP-1-Präparation, die keinem Verfahren gemäß Anspruch 1 unterzogen wurde (N-terminal unmodifizierte MCP-1-Präparation), im Verhältnis 100 : 48 oder höher liegt.

**11.** Rekombinant hergestellte MCP-1-Präparation, in der zumindest 90 % des MCP-1-Proteins als pyroGlu-MCP-1 vorliegen.

**12.** MCP-1-Präparation nach Anspruch 11, bei der das pyroGlu-MCP-1 in nichtglycosylierter Form vorliegt.

$t_0$:

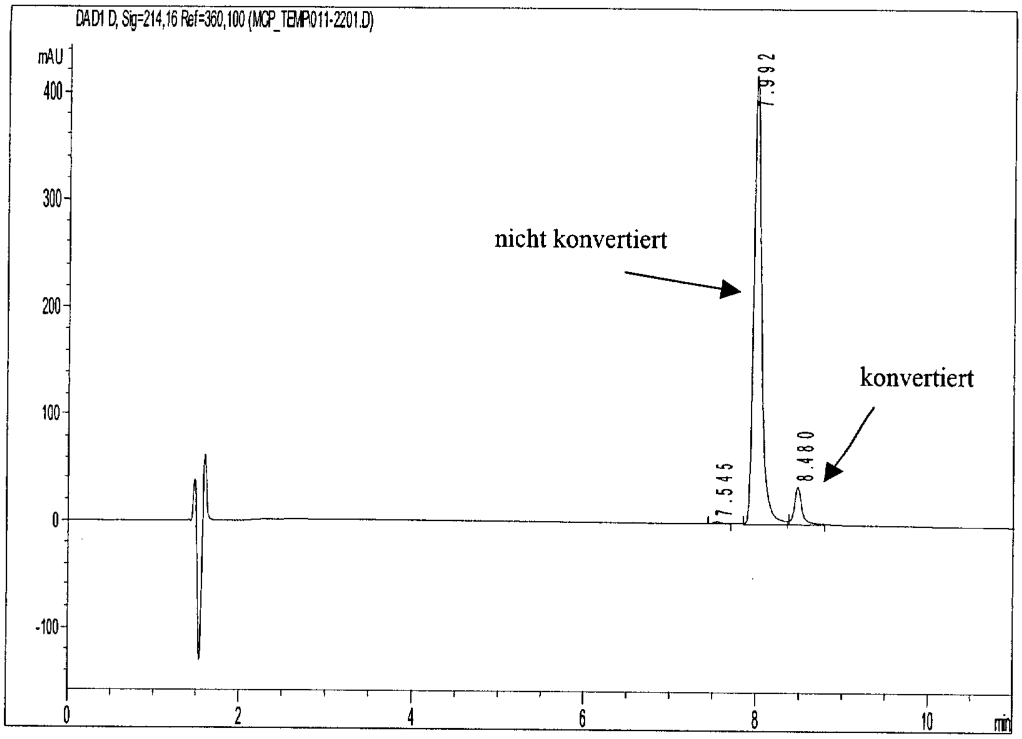
DAD1 D, Sig=214,16 Ref=360,100 (MCP_TEMP\011-2201.D)
mAU
400
300
200
100
0
-100
7.992
7.545
8.480
nicht konvertiert
konvertiert
0
2
4
6
8
10
min

24 h:

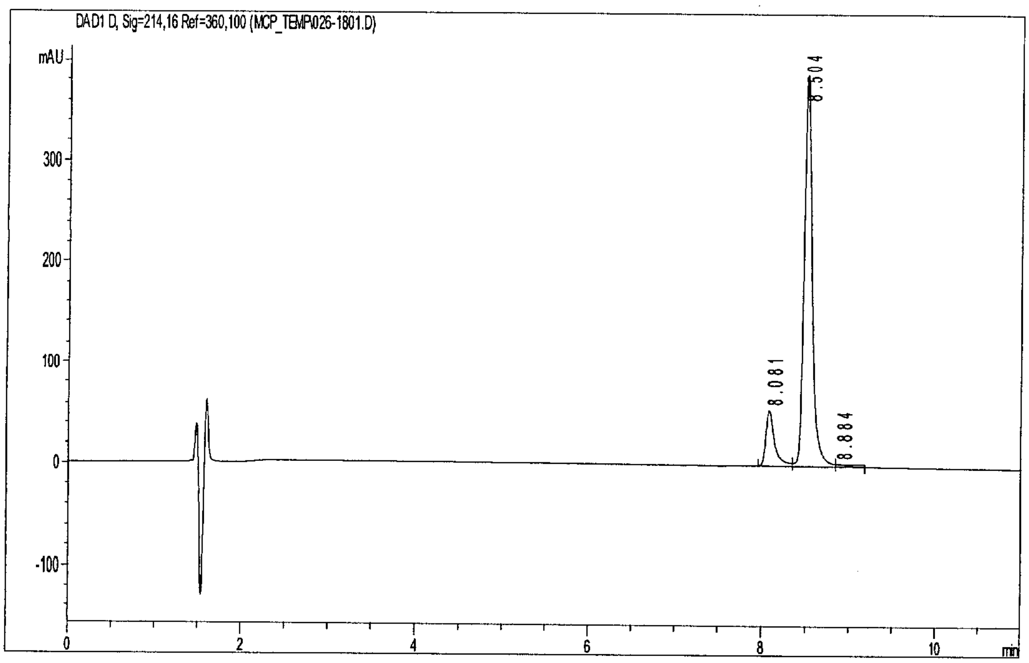
DAD1 D, Sig=214,16 Ref=360,100 (MCP_TEMP\026-1801.D)
mAU
300
200
100
0
-100
8.504
8.081
8.884
0
2
4
6
8
10
min

**FIG. 1**

48 h:

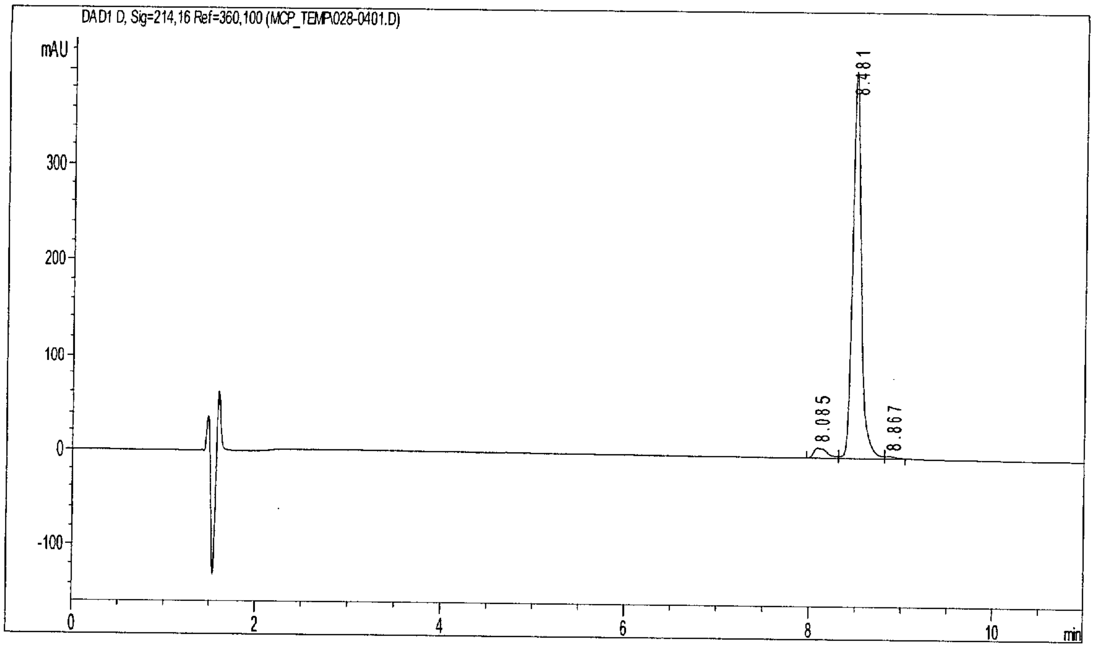


120 h:

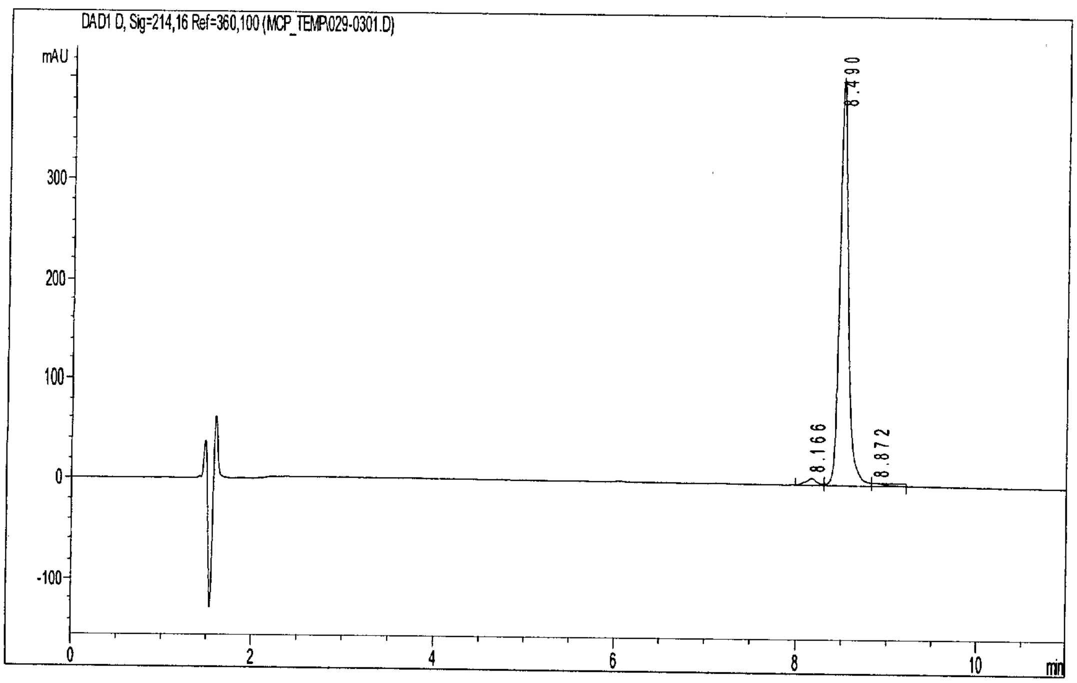


**FIG. 1** (Fortsetzung)

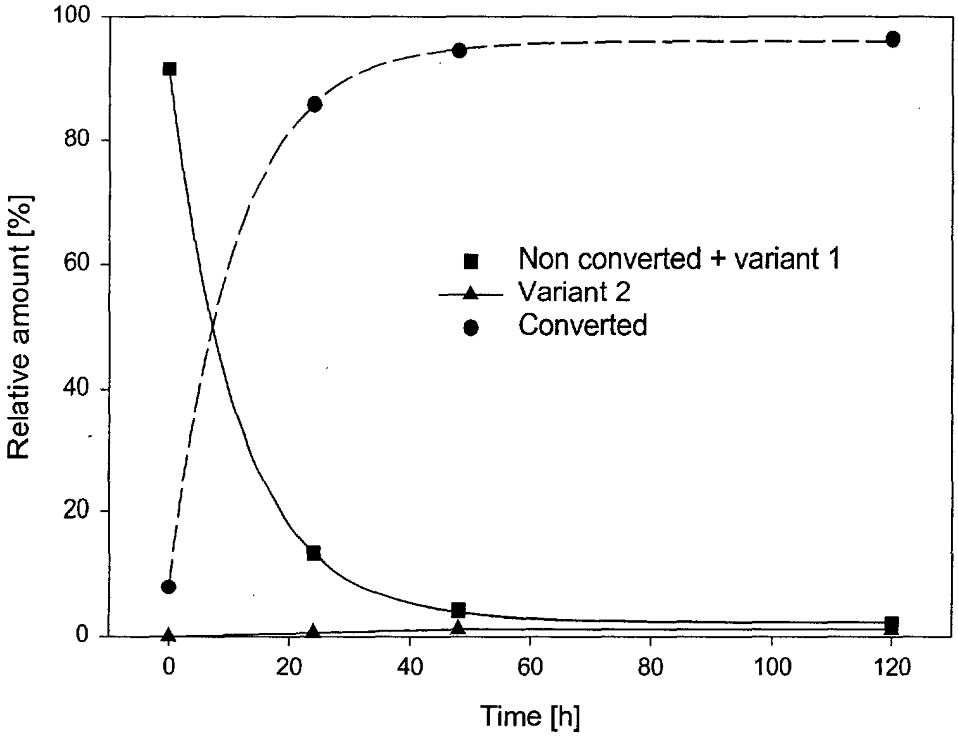
100
80
60
40
20
0
Relative amount [%]
0
20
40
60
80
100
120
Time [h]
Non converted + variant 1
Variant 2
Converted

**FIG. 2**

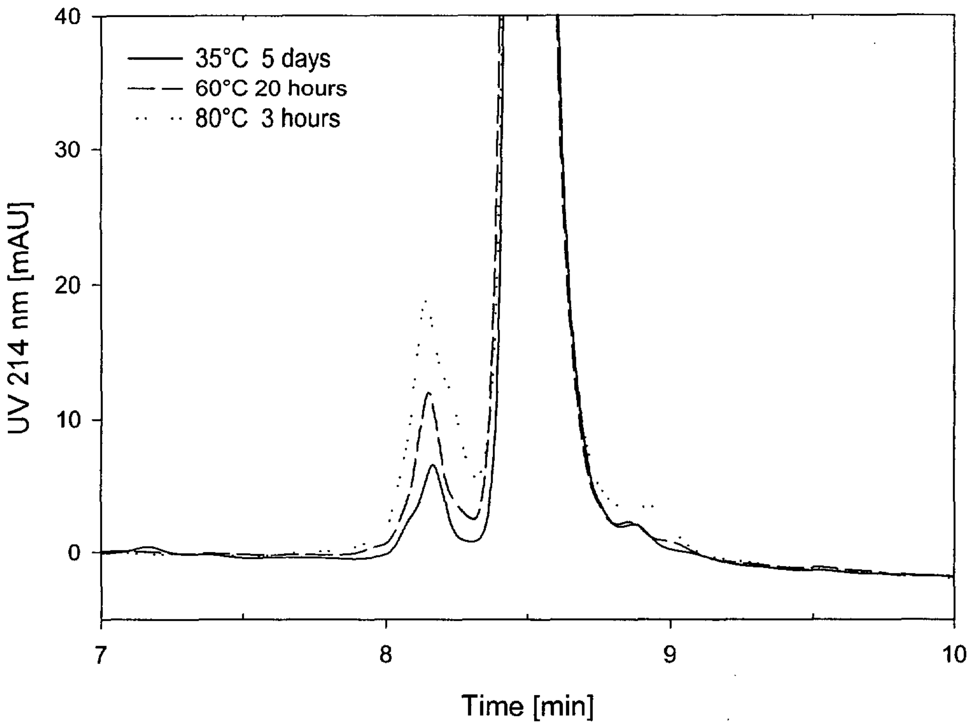
35°C 5 days
60°C 20 hours
80°C 3 hours
UV 214 nm [mAU]
Time [min]
40
30
20
10
0
7
8
9
10

FIG. 3

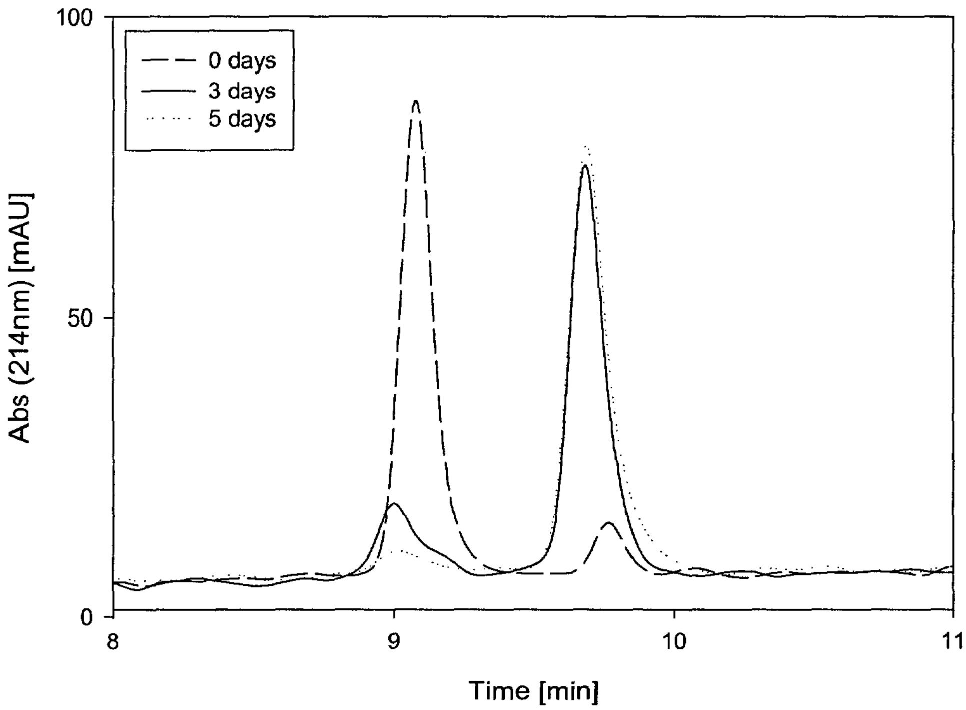


**FIG. 4**

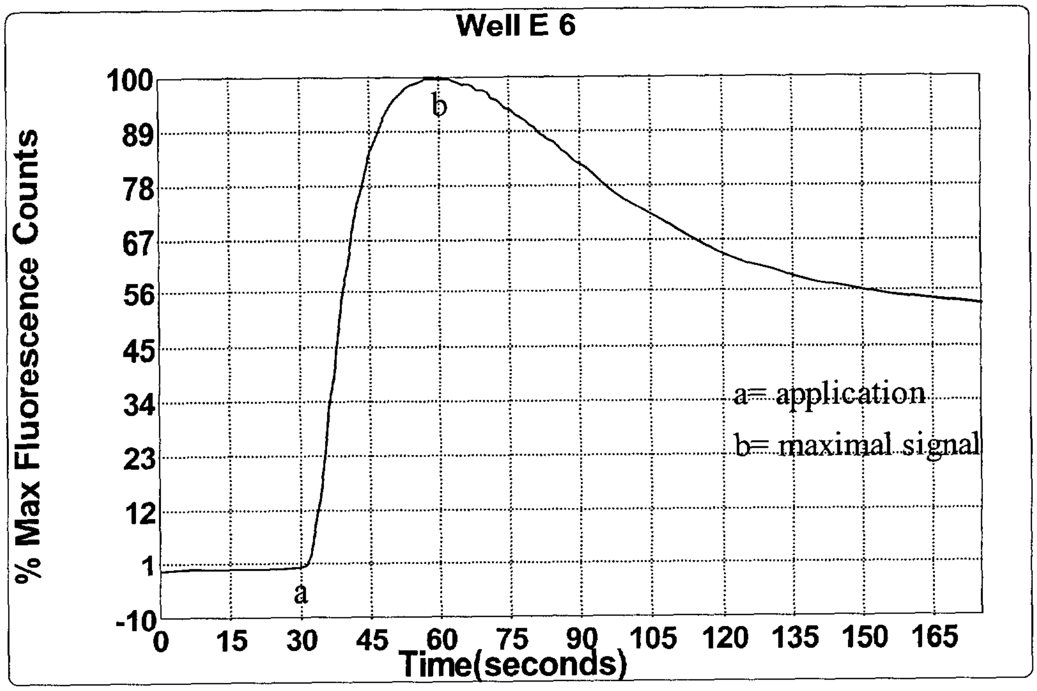
Well E 6
% Max Fluorescence Counts
100
89
78
67
56
45
34
23
12
1
-10
0 15 30 45 60 75 90 105 120 135 150 165
Time(seconds)
a
b
a= application
b= maximal signal

FIG. 5

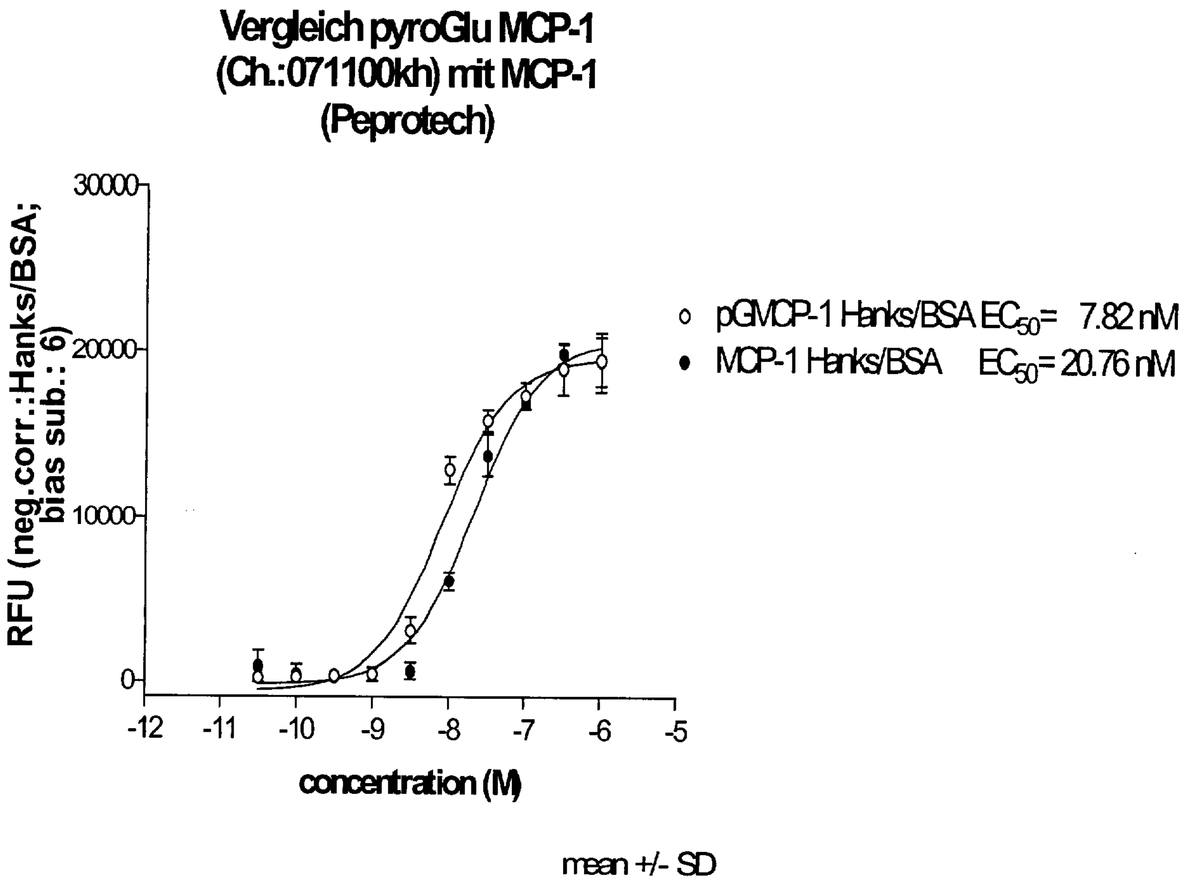
Vergleich pyroGlu MCP-1
(Ch.:071100kh) mit MCP-1
(Peprotech)
RFU (neg.corr.:Hanks/BSA; bias sub.: 6)
30000
20000
10000
0
-12
-11
-10
-9
-8
-7
-6
-5
concentration (M)
pGMCP-1 Hanks/BSA EC50= 7.82 nM
MCP-1 Hanks/BSA EC50= 20.76 nM
mean +/- SD

**FIG. 6**

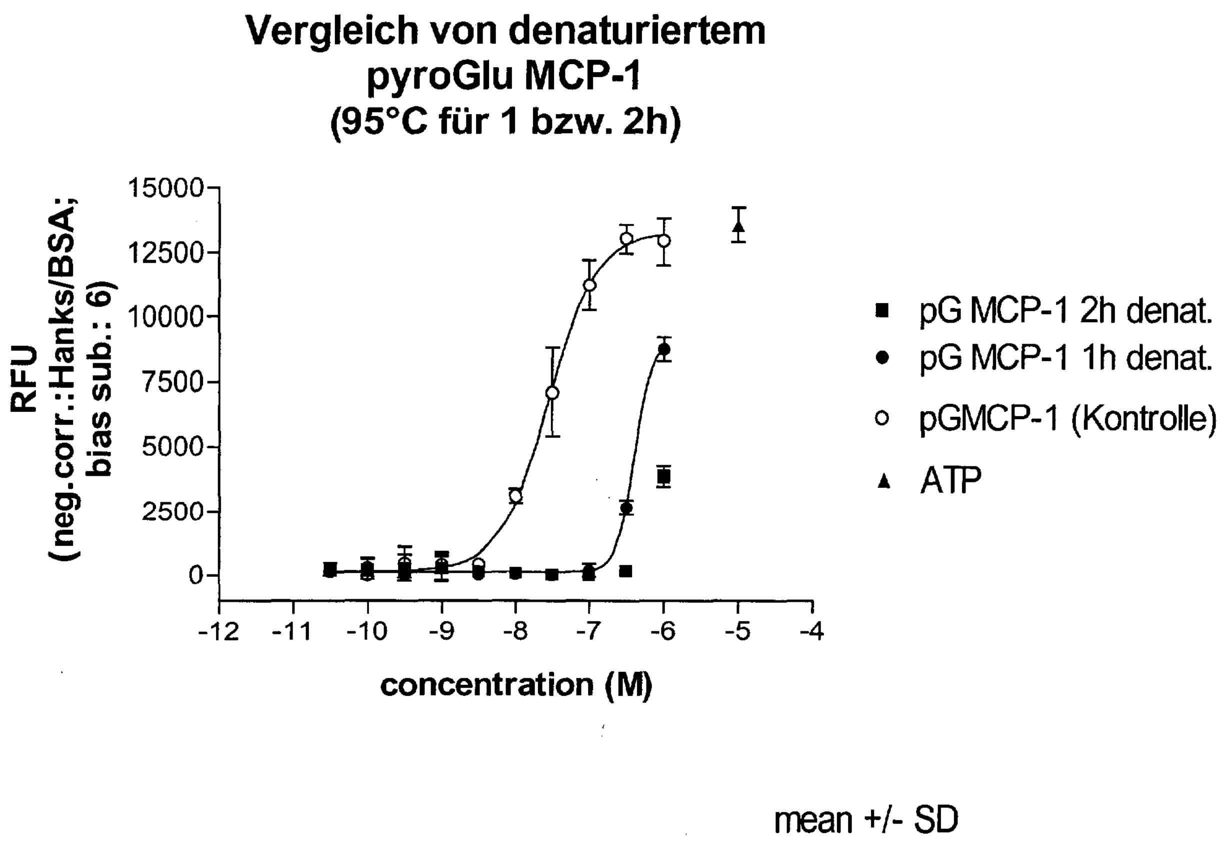
Vergleich von denaturiertem
pyroGlu MCP-1
(95°C für 1 bzw. 2h)
RFU
(neg.corr.:Hanks/BSA;
bias sub.: 6)
15000
12500
10000
7500
5000
2500
0
-12
-11
-10
-9
-8
-7
-6
-5
-4
concentration (M)
pG MCP-1 2h denat.
pG MCP-1 1h denat.
pGMCP-1 (Kontrolle)
ATP
mean +/- SD

FIG. 7

Europäisches Patentamt

# EUROPÄISCHER RECHERCHENBERICHT

Nummer der Anmeldung
EP 03 01 0014

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| X | BLASZCZYK J ET AL.: "Complete Crystal Structure of Monocyte Chemotactic Protein-2, a CC Chemokine that Interacts with Multiple Receptors"<br>BIOCHEMISTRY,<br>Bd. 39, 2000, Seiten 14075-14081, XP002253471<br>* Seite 14075 - Seite 14076 *<br>--- | 5-8, 10-12 | C07K14/52<br>C12N15/19<br>A61K38/19<br>A61P9/00 |
| X | US 6 090 795 A (YOSHIMURA ET AL.)<br>18. Juli 2000 (2000-07-18)<br>* Spalte 1 - Spalte 2; Ansprüche 1-12; Beispiele 1,3 *<br>--- | 5-8, 10-12 | |
| X,D | US 5 714 578 A (APPELLA ETTORE ET AL)<br>3. Februar 1998 (1998-02-03)<br>* das gesamte Dokument, besonders Spalten 1-3, Beispiele III-V, Tabelle VI and Anspruch 1 *<br>--- | 5-8, 10-12 | |
| X | VOSKUIL M ET AL.: "Modulation of collateral artery growth in a porcine hindlimb ligation model using MCP-1"<br>AMERICAN JOURNAL OF PHYSIOLOGY,<br>Bd. 284, Nr. 2(2), April 2003 (2003-04), Seiten H1422-H1428, XP008021581<br>* das ganze Dokument *<br>--- | 9 | RECHERCHIERTE SACHGEBIETE (Int.Cl.7)<br>C07K<br>C12N<br>A61K |
| A,D | VAN COILLIE E ET AL.: "Functional Comparision of Two Human Monocyte Chemotactic Protein-2 Isoforms, Role of the Amino-Terminal Pyroglutamic Acid and Processing by CD26/Dipeptidyl Peptidase IV"<br>BIOCHEMISTRY,<br>Bd. 37, 1998, Seiten 12672-12680, XP002253472<br>* Seite 12673 - Seite 12674 *<br>---<br>-/-- | 1-4 | |

Der vorliegende Recherchenbericht wurde für alle Patentansprüche erstellt

| Recherchenort | Abschlußdatum der Recherche | Prüfer |
|---|---|---|
| DEN HAAG | 4. September 2003 | Schmidt, Harald |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie,übereinstimmendes Dokument

EPO FORM 1503 03.82 (P04C03)

Europäisches Patentamt

# EUROPÄISCHER RECHERCHENBERICHT

Nummer der Anmeldung
EP 03 01 0014

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| A | US 6 383 782 B1 (BARRATT DEREK GRAHAM ET AL) 7. Mai 2002 (2002-05-07)<br>* SEQ ID NO:32 *<br>* Spalte 1; Beispiele 1,2 *<br>--- | | |
| A | ISHII K ET AL.: "Full active baculovirus-expressed human monocyte chemoattractant protein 1 with the intact N-terminus"<br>BIOCHEMICAL AND BIOPHYSICAL RESEARCH COMMUNICATIONS,<br>Bd. 206, Nr. 3, 1995, Seiten 955-961, XP002253473<br>* Seite 956 *<br>----- | | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| DEN HAAG | 4. September 2003 | Schmidt, Harald |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 03 01 0014

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-09-2003

| Im Recherchenbericht angeführtes Patentdokument | Datum der Veröffentlichung | Mitglied(er) der Patentfamilie | Datum der Veröffentlichung |
|---|---|---|---|
| US 6090795 A | 18-07-2000 | US 5532144 A | 02-07-1996 |
| | | AU 5048090 A | 24-08-1990 |
| | | AU 5160390 A | 24-08-1990 |
| | | CA 2006964 A1 | 31-07-1990 |
| | | WO 9008777 A1 | 09-08-1990 |
| | | WO 9008778 A1 | 09-08-1990 |
| | | US 5714578 A | 03-02-1998 |
| US 5714578 A | 03-02-1998 | AU 5048090 A | 24-08-1990 |
| | | CA 2006964 A1 | 31-07-1990 |
| | | WO 9008777 A1 | 09-08-1990 |
| | | AU 5160390 A | 24-08-1990 |
| | | WO 9008778 A1 | 09-08-1990 |
| | | US 5532144 A | 02-07-1996 |
| | | US 6090795 A | 18-07-2000 |
| US 6383782 B1 | 07-05-2002 | AU 8453598 A | 16-02-1999 |
| | | EP 0998564 A1 | 10-05-2000 |
| | | WO 9905279 A1 | 04-02-1999 |

EPO FORM P0461

Für nähere Einzelheiten zu diesem Anhang : siehe Amtsblatt des Europäischen Patentamts, Nr.12/82